(19) Europäisches Patentamt
European Patent Office
Office européen des brevets

(11) Veröffentlichungsnummer: **0 101 996**
**B1**

(12) **EUROPÄISCHE PATENTSCHRIFT**

(45) Veröffentlichungstag der Patentschrift: **16.07.86**

(51) Int. Cl.⁴: **C 07 D 249/08** // A01N43/64

(21) Anmeldenummer: **83107842.3**

(22) Anmeldetag: **09.08.83**

# (54) Verfahren zur Herstellung von E-Isomeren von 1-Cyclohexyl-2-(1,2,4-triazol-1-yl)-1-penten-3-on-Derivaten.

(30) Priorität: **21.08.82 DE 3231205**

(43) Veröffentlichungstag der Anmeldung: **07.03.84 Patentblatt 84/10**

(45) Bekanntmachung des Hinweises auf die Patenterteilung: **16.07.86 Patentblatt 86/29**

(84) Benannte Vertragsstaaten: **BE CH DE FR GB LI**

(56) Entgegenhaltungen:
**EP-A-0 015 387**
**EP-A-0 032 239**
**GB-A-2 046 260**

(73) Patentinhaber: **BAYER AG, Konzernverwaltung RP Patentabteilung, D-5090 Leverkusen 1 Bayerwerk (DE)**

(72) Erfinder: **Reiser, Wolf, Dr., Kiebitzweg 12a, D-5600 Wuppertal 1 (DE)**
Erfinder: **Elbe, Hans-Ludwig, Dr., Dasnöckel 59, D-5600 Wuppertal 11 (DE)**
Erfinder: **Feyen, Peter, Dr., Mozartstrasse 1, D-4020 Mettmann (DE)**

**EP 0 101 996 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von bekannten pflanzenwachstumsregulierend und fungizid wirksamen E-Isomeren von 1-Cyclohexyl-2-(1,2,4-triazol-1-yl)-1-penten-3-on-Derivaten.

Es ist bereits bekannt geworden, dass man 1-Cyclohexyl-2-(1,2,4-triazol-1-yl)-1-penten-3-on-Derivate erhält, wenn man Cyclohexanaldehyd mit entsprechenden Triazolylketonen in Gegenwart eines basischen Katalysators, wie z.B. Piperidinacetat, und in Gegenwart eines inerten organischen Lösungsmittels, insbesondere eines (aromatischen) Kohlenwasserstoffes, bei der Siedetemperatur des jeweiligen Lösungsmittels umsetzt.

Die Isolierung der Endprodukte, und hier insbesondere die Isolierung der verschiedenen Isomeren erfolgt vorzugsweise über ihre Säureadditionssalze (vergleiche EP-OS 15 387).

Dieses Verfahren hat den Nachteil, dass die Herstellung von reinen Isomeren, wie insbesondere der E-Isomeren, in wirtschaftlich ungünstiger Art und Weise verläuft. So erfordert das Freisetzen des jeweiligen reinen Isomeren aus dem entsprechenden Säureadditionssalz mit Hilfe einer Base, wie zum Beispiel Natriumcarbonat, einen Lösungsmittelwechsel und die Ausbeuten an gewünschtem isomeren Produkt sind allgemein nicht befriedigend.

Aus der GB-A-2 046 260 ist die Isomerisierung von Trazolyl-styryl-ketonen durch UV-Bestrahlung bekannt.

Es wurde nun gefunden, dass man die bekannten E-Isomeren von 1-Cyclohexyl-2-(1,2,4-triazol-1-yl)-1-penten-3-on-Derivaten der Formel

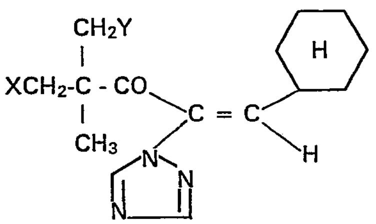

(I)

in welcher

X und Y unabhängig voneinander für Wasserstoff oder Halogen stehen,

erhält, wenn man die Isomerengemische von 1-Cyclohexyl-2-(1,2,4-triazol-1-yl)-1-penten-3-on-Derivaten der Formel

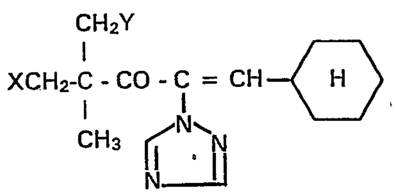

(II)

in welcher

X und Y die oben angegebene Bedeutung haben, mit sekundären Aminen, gegebenenfalls in Gegenwart von Wasser und/oder in Gegenwart eines mit Wasser nicht mischbaren Lösungsmittels umsetzt.

Es ist als überraschend zu bezeichnen, dass sich nach dem erfindungsgemässen Verfahren die Isomerengemische von Verbindungen der Formel (II) in sehr guten Ausbeuten zu den E-Isomeren von Verbindungen der Formel (I) isomerisieren lassen.

Das erfindungsgemässe Verfahren weist somit den Vorteil auf, die E-Isomeren von Verbindungen der Formel (I) in einfacher Weise in sehr guten Ausbeuten erhalten zu können.

Die nach dem erfindungsgemässen Verfahren herstellbaren E-Isomeren von 1-Cyclohexyl-2-(1,2,4-triazol-1-yl)-1-penten-3-on-Derivaten sind durch die Formel (I) allgemein definiert. In dieser Formel stehen X und Y unabhängig voneinander vorzugsweise für Wasserstoff, Fluor, Chlor oder Brom.

Besonders bevorzugt sind diejenigen Verbindungen der Formel (I), in denen X für Wasserstoff steht und Y für Wasserstoff, Fluor oder Chlor steht.

Verwendet man beispielsweise das Isomerengemisch von 1-Cyclohexyl-4,4-dimethyl-2-(1,2,4-triazol-1-yl)-1-penten-3-on und Piperidin als Ausgangsstoffe, so kann der Reaktionsablauf durch das folgende Formelschema wiedergegeben werden:

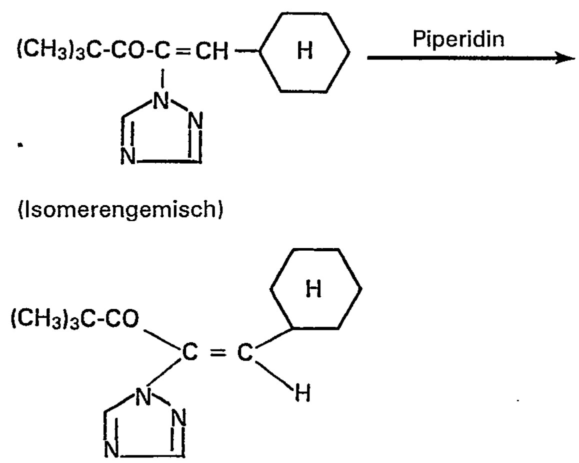


(Isomerengemisch)

(E-Isomeres)

Die bei der Durchführung des erfindungsgemässen Verfahrens als Ausgangsstoffe zu verwendenden Isomerengemische von 1-Cyclohexyl-2-(1,2,4-triazol-1-yl)-penten-3-on-Derivaten sind durch die Formel (II) allgemein definiert. In dieser Formel stehen X und Y vorzugsweise für die Reste, die bereits im Zusammenhang mit der Beschreibung der erfindungsgemäss herstellbaren Stoffe der Formel (I) vorzugsweise für diese Substituenten genannt wurden.

Die Isomerengemische der Formel (II) sind bekannt (vergleiche EP-OS 15 387).

Die erfindungsgemässe Umsetzung erfolgt mit sekundären Aminen. Hierbei können alle üblicherweise verwendbaren sekundären Amine eingesetzt werden. Vorzugsweise in Frage kommen Piperidin, Pyrrolidin, Dimethylamin oder Diethylamin.

Die erfindungsgemässe Umsetzung kann gegebenenfalls in Gegenwart von Wasser und/oder eines mit Wasser nicht mischbaren Lösungsmittels durchgeführt werden. Hierzu gehören vorzugsweise aliphatische, cycloaliphatische und aromatische Kohlenwasserstoffe, wie beispielsweise Cyclohexan, Petrolether, Toluol, Isododecan, Isooctan oder Ligroin.

Die erfindungsgemässe Umsetzung wird vorzugsweise bei Temperaturen zwischen —30 und +30 °C durchgeführt.

Bei der Durchführung der erfindungsgemässen Umsetzung setzt man auf 1 Mol Isomerengemisch der Formel (II) vorzugsweise eine katalytische Menge oder auch bis zu 0,5 Mol an sekundärem Amin ein. Die Isolierung der Endprodukte erfolgt in allgemein üblicher Art und Weise.

Die erfindungsgemäss herstellbaren Stoffe zeichnen sich bekanntermassen durch gute pflanzenwachstumsregulierende und fungizide Wirksamkeit aus (vergleiche EP-OS 15 387). Ausserdem können sie als Zwischenprodukte zur Herstellung von weiteren pflanzenwachstumsregulierenden und fungiziden Verbindungen eingesetzt werden, z.B. durch übliche Reduktion der Ketogruppe (vergleiche EP-OS 15 387).

Das erfindungsgemässe Verfahren sei anhand der folgenden Herstellungsbeispiele erläutert:

*Herstellungsbeispiele*

*Beispiel 1*

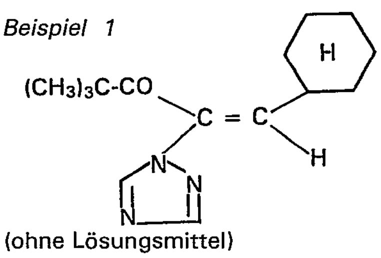

(ohne Lösungsmittel)

Zu 130 g (0,5 Mol) des Isomerengemisches von 1-Cyclohexyl-4,4-dimethyl-2-(1,2,4-triazol-1-yl)-1-penten-3-on (bestehend aus 29% E-Isomerem, 69% Z-Isomeren und 2% Isomeren mit verschobener Doppelbindung) werden bei Raumtemperatur unter kräftigem Rühren 17 g (0,2 Mol) Piperidin getropft, wobei das Reaktionsgemisch unter Erwärmung zu einem Kristallbrei erstarrt. Man lässt das Reaktionsgemisch 2 Stunden stehen, versetzt dann mit 100 ml n-Pentan und verrührt. Nach Abkühlung wird der feinkristalline Feststoff abgetrennt und mit wenig kaltem n-Pentan aminfrei gewaschen. Man erhält 100 g (77% der Theorie) reines E-Isomeres des 1-Cyclohexyl-4,4-dimethyl-2-(1,2,4-triazol-1-yl)-1-penten-3-ons vom Schmelzpunkt 97 - 98 °C.

(in Gegenwart von Wasser)

130 g (0,5 Mol) des Isomerengemisches von 1-Cyclohexyl-4,4-dimethyl-2-(1,2,4-triazol-1-yl)-1-penten-3-on (bestehend aus 29% E-Isomerem, 69% Z-Isomerem und 2% Isomeren mit verschobener Doppelbindung) werden unter kräftigem Rühren zu einer Lösung von 20 ml 30%igem wässrigem Dimethylamin und 100 ml Wasser gegeben. Die zunächst ölige Emulsion verfestigt sich innerhalb von etwa 2 Stunden und lässt sich danach abnutschen. Der Rückstand wird mehrmals mit Wasser gewaschen, getrocknet und in 100 ml n-Pentan aufgenommen. Der feinkristalline Niederschlag wird abgesaugt und getrocknet. Man erhält 98 g (75,4% der Theorie) reines E-Isomeres des 1-Cyclohexyl-4,4-dimethyl-2-(1,2,4-triazol-1-yl)-1-penten-3-ons vom Schmelzpunkt 98 °C.

**Patentansprüche**

1. Verfahren zur Herstellung von E-Isomeren von 1-Cyclohexyl-2-(1,2,4-triazol-1-yl)-1-penten-3-on-Derivaten der Formel

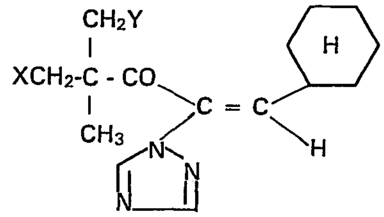

(I)

in welcher

X und Y unabhängig voneinander für Wasserstoff oder Halogen stehen,

dadurch gekennzeichnet, dass man Isomerengemische von 1-Cyclohexyl-2-(1,2,4-triazol-1-yl)-1-penten-3-on-Derivaten der Formel

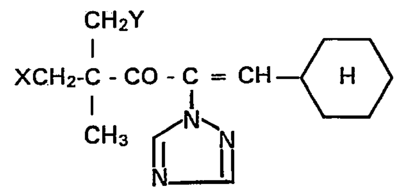

(II)

in welcher

X und Y die oben angegebene Bedeutung haben, mit sekundären Aminen, gegebenenfalls in Gegenwart von Wasser und/oder in Gegenwart eines mit Wasser nicht mischbaren Lösungsmittels umsetzt.

2. Verfahren gemäss Anspruch 1, dadurch gekennzeichnet, dass man als sekundäres Amin, Piperidin, Pyrrolidin, Dimethylamin oder Diethylamin einsetzt.

3. Verfahren gemäss Anspruch 1, dadurch gekennzeichnet, dass man als mit Wasser nicht mischbare Lösungsmittel aliphatische, cycloaliphatische oder aromatische Kohlenwasserstoffe einsetzt.

4. Verfahren gemäss Anspruch 1, dadurch gekennzeichnet, dass man die Umsetzung bei Temperaturen zwischen —30 °C und +30 °C durchführt.

5. Verfahren gemäss Anspruch 1, dadurch gekennzeichnet, dass man auf 1 Mol an Isomerengemisch der Formel (II) von einer katalytischen Menge bis zu 0,5 Mol an sekundärem Amin einsetzt.

**Claims**

1. Process for the preparation of E-isomers of 1-cyclohexyl-2-(1,2,4-triazol-1-yl)-1-penten-3-one derivatives of the formula

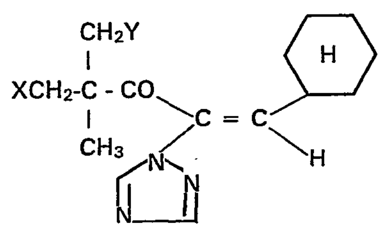

(I)

in which

X and Y independently of one another represent hydrogen or halogen,

characterised in that mixtures of isomers of 1-cyclohexyl-2-(1,2,4-triazol-1-yl)-1-penten-3-one derivatives of the formula

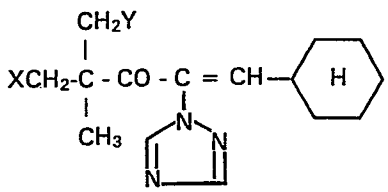

(II)

in which

X and Y have the meaning indicated above,

are reacted with secondary amines, if appropriate in the presence of water and/or in the presence of a water-immiscible solvent.

2. Process according to claim 1, characterised in that the secondary amine employed is piperidine, pyrrolidine, dimethylamine or diethylamine.

3. Process according to claim 1, characterised in that aliphatic, cycloaliphatic or aromatic hydrocarbons are employed as the water-immiscible solvents.

4. Process according to claim 1, characterised in that the reaction is carried out at temperatures between —30 and + 30°C.

5. Process according to claim 1, characterised in that from a catalytic amount to 0.5 mol of secondary amine is employed per mol of a mixture of isomers of the formula (II).

**Revendications**

1. Procédé de préparation des isomères E de dérivés de la 1-cyclohexyl-2-(1,2,4-triazole-1-yl)-1-pentène-3-one, de formule

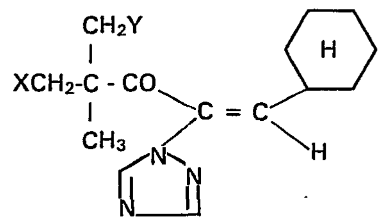

(I)

dans laquelle

X et Y représentent chacun, indépendamment l'un de l'autre, l'hydrogène ou un halogène,

caractérisé en ce que l'on fait réagir des mélanges d'isomères de dérivés de la 1-cyclohexyl-2-(1,2,4-triazole-1-yl)-1-pentène-3-one de formule

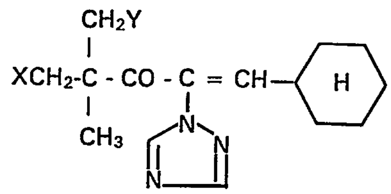

(II)

dans laquelle

X et Y ont les significations indiquées ci-dessus,

avec des amines secondaires, éventuellement en présence d'eau et/ou en présence d'un solvant non miscible à l'eau.

2. Procédé selon la revendication 1, caractérisé en ce que l'on utilise en tant qu'amine secondaire la pipéridine, la pyrrolidine, la diméthylamine ou la diéthylamine.

3. Procédé selon la revendication 1, caractérisé en ce que l'on utilise en tant que solvants non miscibles à l'eau des hydrocarbures aliphatiques, cycloaliphatiques ou aromatiques.

4. Procédé selon la revendication 1, caractérisé en ce que l'on effectue la réaction à des températures allant de —30°C jusqu'à +30°C.

5. Procédé selon la revendication 1, caractérisé en ce que l'on utilise l'amine secondaire en quantité catalytique ou en quantité allant jusqu'à 0,5 mole pour 1 mole du mélange d'isomères de formule II.